# EUROPÄISCHE PATENTANMELDUNG

(11) **EP 4 049 597 A1**
(43) Veröffentlichungstag der Anmeldung: **31.08.2022**
(21) Anmeldenummer: 22152974.6
(22) Anmeldetag: 24.01.2022
(51) Int. Cl.: A61B 17/29, A61B 17/00

(54) **CHIRURGISCHES INSTRUMENT ZUR MINIMALINVASIVEN CHIRURGIE**

(30) Priorität: 26.02.2021 DE 102021104642
(71) Anmelder: avateramedical GmbH, 07745 Jena (DE)
(72) Erfinder: MÄMPEL, Jörg, 98693 Ilmenau (DE)
(74) Vertreter: Schaumburg und Partner Patentanwälte mbB

(57) **Zusammenfassung**

Ein chirurgisches Instrument (10, 10', 10") zur minimalinvasiven Chirurgie umfasst einen Instrumentenschaft (12) mit einem distalen Ende (14), mit dem ein Instrumentenkopf (20) über mindestens ein Gelenk schwenkbar verbunden ist. Weiterhin umfasst das chirurgische Instrument (10, 10', 10") einen Endeffektor (22), der um seine Längsachse (38) drehbar im Instrumentenkopf (20) gelagert ist. Der Endeffektor (22) ist mit Hilfe einer zum Instrumentenschaft (12) koaxialen Antriebswelle (32) drehbar. Das chirurgische Instrument (10, 10', 10") umfasst eine flexible Hohlwelle (42, 100, 200, 300,400, 500, 600, 700, 800, 900, 1000), die eine Drehbewegung von einem Ende der Antriebswelle (32) zum Endeffektor (22) zumindest im Bereich des Gelenks überträgt. Die flexible Hohlwelle (42, 100, 200, 300, 400, 500, 600, 700, 800, 900, 1000) umfasst mehrere in Längsrichtung der Hohlwelle (42, 100, 200, 300, 400, 500, 600, 700, 800, 900, 1000) nebeneinander angeordnete Federscheiben (54), die durch in der Wandung der Hohlwelle (42, 100, 200, 300, 400, 500, 600, 700, 800, 900, 1000) radial erstreckende Schlitze (60) entlang eines Teils des Umfangs der Hohlwelle (42, 100, 200, 300, 400, 500, 600, 700, 800, 900, 1000) voneinander getrennt sind. In Längsrichtung der Hohlwelle (42, 100, 200, 300, 400, 500, 600, 700, 800, 900, 1000) sind benachbarte Federscheiben (54) durch mindestens zwei Verbindungstege (58) miteinander verbunden. Weiterhin sind in Längsrichtung der Hohlwelle (42, 100, 200, 300, 400, 500, 600, 700, 800, 900, 1000) benachbarte Verbindungsstege (58) jeweils durch Federstege (62) einer Federscheibe (54) verbunden.

## Beschreibung

Die Erfindung betrifft ein chirurgisches Instrument zur minimalinvasiven Chirurgie, das einen Instrumentenschaft mit einem distalen Ende hat. An dem distalen Ende ist ein Instrumentenkopf über mindestens ein Gelenk schwenkbar mit dem Instrumentenschaft verbunden. Ferner umfasst das chirurgische Instrument einen Endeffektor, der um seine Längsachse drehbar im Instrumentenkopf gelagert ist und mit Hilfe einer zum Instrumentenschaft koaxialen Antriebswelle drehbar ist. Ein solches Instrument zur minimalinvasiven Chirurgie ist beispielsweise aus dem Dokument EP 2 377 477 A1 bekannt.

Aus dem Dokument EP 3 025 667 A1 ist eine Vorrichtung zur robotergestützten Chirurgie bekannt, bei der ein chirurgisches Instrument mit Hilfe eines Manipulatorarms eines Manipulators geführt und betätigt wird. Hierzu ist am distalen Ende des Manipulatorarms eine Koppeleinheit mit Antriebselementen und Übertragungsmitteln angeordnet, mit der eine am proximalen Ende eines Instrumentenschafts des chirurgischen Instruments angeordnete Instrumenteneinheit über eine Sterilschleuse koppelbar ist.

Diese chirurgischen Instrumente zur minimalinvasiven Chirurgie haben in der Regel einen starren Instrumentenschaft, mit dessen Hilfe ein an einem distalen Ende des Instrumentenschafts angeordneter Endeffektor durch einen Trokar in den Körper eines Patienten einführbar ist. Um trotz des starren Instrumentenschafts eine flexible Positionierung und Steuerung des Endeffektors im Operationsfeld zu ermöglichen, haben die chirurgischen Instrumente einen flexiblen Instrumentenkopf, der an einem Gelenk abgewinkelt werden kann. Um eine noch größere Bewegungsfreiheit zu haben, kann zusätzlich der Endeffektor drehbar in dem Instrumentenkopf gelagert sein. Um insbesondere bei abgewinkeltem Instrumentenkopf eine Drehbewegung zum Endeffektor zu übertragen und dabei insbesondere den Bereich des Gelenks zu überbrücken, ist eine flexible mechanische Verbindung zwischen einer Antriebswelle und dem Endeffektor notwendig. Aus dem Dokument EP 2 413 819 A1 ist ein chirurgisches Instrument mit einem flexiblen Metallbalg zur Übertragung einer Drehbewegung an einen Endeffektor bekannt.

Das aus dem Dokument EP 2 377 477 A1 bekannte chirurgische Instrument hat einen beweglichen Instrumentenkopf, wobei mit Hilfe einer Spiralfeder auch bei abgewinkeltem Instrumentenkopf eine Drehung mechanisch von einer Antriebswelle auf einen Endeffektor übertragbar ist. Die Spiralfeder hat den Nachteil, dass sie eine geringe Torsionssteifigkeit hat, so dass bei Einleitung einer Drehung zunächst eine Spannung in der Spiralfeder aufgebaut werden muss, bevor ein Drehmoment von der Antriebswelle auf den Endeffektor übertragen werden kann. Das hat zur Folge, dass eine Steuerung und Positionierung des Endeffektors mit einer Verzögerung geschieht und ruckartige Bewegungen hervorgerufen werden können. Das erschwert die präzise Positionierung und Steuerung des Endeffektors in einem Operationsfeld.

Es ist Aufgabe der vorliegenden Erfindung, ein chirurgisches Instrument zur minimalinvasiven Chirurgie anzugeben, dass einen einfachen Aufbau hat und eine besonders robuste Steuerung eines Endeffektors erlaubt. Insbesondere ist es Aufgabe der Erfindung, sowohl bei abgewinkelter als auch bei gerader Position eines Instrumentenkopfs eine präzise Drehung des Endeffektors zu ermöglichen.

Diese Aufgabe wird durch ein chirurgisches Instrument mit den Merkmalen des Anspruchs 1 gelöst. Vorteilhafte Weiterbildungen sind in den abhängigen Ansprüchen angegeben.

Das chirurgische Instrument zur minimalinvasiven Chirurgie umfasst nach Anspruch 1 einen Instrumentenschaft mit einem distalen Ende, mit dem ein Instrumentenkopf über mindestens ein Gelenk schwenkbar verbunden ist. Weiterhin umfasst das chirurgische Instrument einen Endeffektor, der um seine Längsachse drehbar im Instrumentenkopf gelagert ist. Der Endeffektor ist mit Hilfe einer zum Instrumentenschaft koaxialen Antriebswelle drehbar. Das chirurgische Instrument ist an einem proximalen Ende mit einer manuellen Betätigungseinrichtung oder mit einem Robotersystem zur Betätigung zumindest des Instrumentenschafts und der Antriebswelle verbindbar. Ferner umfasst das chirurgische Instrument eine flexible Hohlwelle, die eine Drehbewegung von einem Ende der Antriebswelle zum Endeffektor zumindest im Bereich des Gelenks überträgt. Die flexible Hohlwelle umfasst mehrere in Längsrichtung der Hohlwelle nebeneinander angeordnete Federscheiben, die durch in der Wandung der Hohlwelle radial erstreckende Schlitze entlang eines Teils des Umfangs der Hohlwelle voneinander getrennt sind. In Längsrichtung der Hohlwelle sind benachbarte Federscheiben durch mindestens zwei Verbindungstege miteinander verbunden. Weiterhin sind in Längsrichtung der Hohlwelle benachbarte Verbindungsstege jeweils durch Federstege einer Federscheibe verbunden.

Die erfindungsgemäße flexible Hohlwelle hat den Vorteil einer besonders präzisen Übertragung eines Drehmoments von der Antriebswelle zum Endeffektor, insbesondere durch eine hohe Torsionssteifigkeit und Biegeelastizität der flexiblen Hohlwelle. Dies gilt sowohl in einem geraden Zustand des Instrumentenkopfs als auch in einem abgewinkelten Zustand des Instrumentenkopfs. Außerdem wird durch die erfindungsgemäße flexible Hohlwelle eine besonders robuste Verbindung zwischen der Antriebswelle und dem Endeffektor erreicht.

Es ist vorteilhaft, wenn die Federscheiben in einem nicht abgewinkelten Zustand des Instrumentenkopfs jeweils in einer orthogonal zur Längsachse der Hohlwelle verlaufenden Ebene parallel zueinander angeordnet sind. Dadurch wird erreicht, dass die Hohlwelle besonders flexibel ist und im Zentrum der Hohlwelle weitere Verbindungselemente hindurchführbar sind.

Weiterhin ist es vorteilhaft, wenn in einem nicht abgewinkelten Zustand des Instrumentenkopfs die vier Federstege, die an einen Verbindungssteg angrenzen, in parallelen Ebenen verlaufen. Dadurch wird eine besonders stabile Bauweise der Hohlwelle erreicht. Alternativ ist es vorteilhaft, wenn in einem nicht abgewinkelten Zustand des Instrumentenkopfs die vier Federstege, die an einen Verbindungssteg angrenzen, zusammen mit dem Verbindungssteg eine X-Form bilden. Dadurch wird erreicht, dass die Hohlwelle eine besonders hohe Biegeelastizität hat und einen großen maximalen Biegewinkel aufweist.

Es ist vorteilhaft, wenn die benachbarten Verbindungsstege, die zwei nebeneinanderliegende Federscheiben verbinden, entlang eines Kreisbogens am Umfang der Hohlwelle gleichmäßig voneinander beabstandet sind. Somit sind bei einer Anzahl von zwei Verbindungsstegen die Verbindungsstege zueinander um 180° um die Längsachse der Hohlwelle versetzt, bei drei Verbindungsstegen die benachbarten Verbindungsstege um 120° und bei vier Verbindungsstegen um 90° zueinander versetzt. Dadurch werden eine besonders hohe Torsionssteifigkeit und eine besonders gleichmäßige Übertragung der Drehung der Antriebswelle an den Endeffektor erreicht, insbesondere wenn die Hohlwelle gebogen ist. Besonders bevorzugt ist es, wenn zwei nebeneinanderliegende Federscheiben mit Hilfe von zwei Verbindungsstegen verbunden sind.

Es ist bevorzugt, wenn die Verbindungsstege einer ersten Ebene orthogonal zur Längsachse der Hohlwelle jeweils zu Verbindungsstegen einer benachbarten zweiten Ebene um 90° um die Längsachse der Hohlwelle versetzt sind. Dadurch werden eine besonders hohe Biegeelastizität und Flexibilität der Hohlwelle erreicht.

Es ist besonders vorteilhaft, wenn die Verbindungsstege um die Längsachse der Hohlwelle so zueinander versetzt angeordnet sind, dass sie in Längsrichtung der Hohlwelle einer Helix-Form folgen. Dadurch wird eine besonders gleichmäßige Drehmomentübertragung unabhängig vom Biegewinkel der Hohlwelle erreicht.

Weiterhin ist es vorteilhaft, wenn die Verbindungsstege einer ersten Ebene jeweils zu Verbindungsstegen einer benachbarten zweiten Ebene insbesondere um 20° bis 89° oder um 91° bis 110° um die Längsachse der Hohlwelle versetzt sind. Alternativ ist es vorteilhaft, wenn die Verbindungstege einer ersten Ebene jeweils zu Verbindungsstegen einer benachbarten zweiten Ebene um 90° um die Längsachse der Hohlwelle versetzt sind und jeweils zu Verbindungsstegen einer übernächsten dritten Ebene insbesondere um 2° bis 40° um die Längsachse der Hohlwelle versetzt sind. Dadurch wird eine besonders gleichmäßige Drehmomentübertragung unabhängig vom Biegewinkel der Hohlwelle erreicht.

Es ist besonders vorteilhaft, wenn sich der Querschnitt eines Federstegs ausgehend von der Mitte eines Federstegs zwischen zwei Verbindungsstegen zu den jeden der Verbindungsstege hin vergrößert. Dadurch wird erreicht, dass bei Biegung der Hohlwelle Spannungen in der Hohlwelle besonders gut verteilt und somit reduziert werden.

Es ist vorteilhaft, wenn der Übergang zwischen zwei in Längsrichtung der Hohlwelle benachbarten Federstegen im Bereich eines Verbindungsstegs rund, insbesondere teilkreisförmig oder parabelförmig, oder eckig, insbesondere dreieckig oder viereckig, ist. Dadurch wird erreicht, dass bei einem Biegen der in Längsrichtung benachbarten Federstege Spannung in der Hohlwelle besonders gut verteilt und somit reduziert werden.

Es ist vorteilhaft, wenn die flexible Hohlwelle bei Rotation um die Längsachse der Hohlwelle torsionssteif und gleichzeitig biegeelastisch bei Abwinklung des Instrumentenkopf ist. Dadurch wird erreicht, dass die Hohlwelle sowohl in einem geraden Zustand als auch in einem abgewinkelten Zustand eine Drehung besonders gut übertragen kann.

Es ist vorteilhaft, wenn der Instrumentenkopf in einem maximal abgewinkelten Zustand zur Längsachse des Instrumentenschafts, entsprechend einer maximalen Biegung der flexiblen Hohlwelle, einen Winkel in einem Bereich von 60° bis 90°, insbesondere von 80°, hat. Dadurch wird erreicht, dass der Endeffektor des chirurgischen Instruments besonders flexibel positionierbar und einsetzbar ist.

Es ist besonders vorteilhaft, wenn die flexible Hohlwelle so ausgebildet ist, dass nebeneinanderliegende Federscheiben in einem maximal abgewinkelten Zustand des Instrumentenkopfs zur Längsachse des Instrumentenschafts, entsprechend einer maximalen Biegung derflexiblen Hohlwelle, an der bezüglich der Biegerichtung inneren Hohlwellenseite gerade in Anlage kommen oder noch voneinander beabstandet sind. Dadurch wird erreicht, dass die Hohlwelle unabhängig von ihrer Biegung eine Drehung besonders präzise von der Antriebswelle an den Endeffektor überträgt.

Es ist vorteilhaft, wenn die Schlitze zwischen nebeneinanderliegenden Federscheiben frei sind. Insbesondere sind die Schlitze frei von elastischen Materialien oder Werkstoffen, beispielsweise frei von Silikon. Dadurch wird erreicht, dass die Hohlwelle besonders flexibel ist und besonders einfach herzustellen ist.

Es ist vorteilhaft, wenn entlang der Längsachse des Instrumentenschafts zumindest ein mechanisches und/oder elektrisches Verbindungselement angeordnet und durch die flexible Hohlwelle hindurchgeführt ist und das mit dem Endeffektor verbindbar ist. Dadurch wird erreicht, dass das chirurgische Instrument mit verschiedenen Funktionen ausgestattet werden kann und somit besonders flexibel einsetzbar ist.

Es ist vorteilhaft, wenn das chirurgische Instrument einen zum Instrumentenschaft koaxialen Innenschaft zum Abwinkeln des Instrumentenkopfs mit Hilfe des Gelenks umfasst, wobei der Innenschaft in Längsrichtung des Instrumentenschafts verschiebbar ist und die Antriebswelle gegenüber dem Instrumentenschaft drehbar ist. Weiterhin ist es vorteilhaft, wenn das chirurgische Instrument an einem proximalen Ende mit einer manuellen Betätigungseinrichtung oder mit einem Robotersystem zur Betätigung des Innenschafts verbindbar ist. Dadurch wird erreicht, dass das chirurgische Instrument besonders einfach zu bedienen beziehungsweise zu steuern ist.

Es ist vorteilhaft, wenn der Endeffektor in dem Instrumentenkopf mit Hilfe eines Kugellagers drehbar gelagert ist. Dadurch wird erreicht, dass der Endeffektor besonders einfach im Instrumentenkopf drehbar ist.

Darüber hinaus ist es vorteilhaft, wenn die flexible Hohlwelle einstückig ausgeführt ist. Dadurch wird eine besonders robuste Bauweise der Hohlwelle erreicht.

Weiterhin ist es vorteilhaft, wenn die flexible Hohlwelle aus Stahl, insbesondere Edelstahl, aus Titan, oder aus einem Kunststoff gefertigt ist. Dadurch wird erreicht, dass die Hohlwelle besonders einfach hergestellt und/oder an unterschiedliche chirurgische Instrumente und chirurgische Einsatzgebiete angepasst werden kann.

Weitere Merkmale und Vorteile ergeben sich aus der folgenden Beschreibung, welche in Verbindung mit den beigefügten Figuren Ausführungsbeispiele näher erläutert.

Es zeigen:
- Figur 1: eine Seitenansicht eines chirurgischen Instruments zur minimalinvasiven Chirurgie,
- Figur 2: einen Längsschnitt des chirurgischen Instruments nach Figur 1 entlang der Mittelebene,
- Figur 3: eine Seitenansicht des chirurgischen Instruments nach Figur 1 mit einem abgewinkelten Instrumentenkopf mit Endeffektor,
- Figur 4: eine Schnittzeichnung des chirurgischen Instruments nach Figur 2 mit einem abgewinkelten Instrumentenkopf mit Endeffektor,
- Figur 5: eine Detailansicht einer Anordnung mit einer flexiblen Hohlwelle gemäß einer ersten Ausführungsform in einem geraden Zustand,
- Figur 6: eine Detailansicht der Anordnung mit der flexiblen Hohlwelle nach Figur 5 in einem gebogenen Zustand,
- Figur 7: eine perspektivische Darstellung einer flexiblen Hohlwelle gemäß einer zweiten Ausführungsform,
- Figur 8: eine Schnittdarstellung der flexiblen Hohlwelle nach Figur 7,
- Figur 9: eine perspektivische Darstellung einer flexiblen Hohlwelle gemäß einer dritten Ausführungsform,
- Figur 10: eine Schnittdarstellung der flexiblen Hohlwelle nach Figur 9,
- Figur 11: eine perspektivische Darstellung einer flexiblen Hohlwelle gemäß einer vierten Ausführungsform,
- Figur 12: eine Schnittdarstellung der flexiblen Hohlwelle nach Figur 11,
- Figur 13: eine perspektivische Darstellung einer flexiblen Hohlwelle gemäß einer fünften Ausführungsform,
- Figur 14: eine Schnittdarstellung der flexiblen Hohlwelle nach Figur 13,
- Figur 15: eine perspektivische Darstellung einer flexiblen Hohlwelle gemäß einer sechsten Ausführungsform,
- Figur 16: eine Schnittdarstellung der flexiblen Hohlwelle nach Figur 15,
- Figur 17: eine perspektivische Darstellung einer flexiblen Hohlwelle gemäß einer siebten Ausführungsform,
- Figur 18: eine Schnittdarstellung der flexiblen Hohlwelle nach Figur 17,
- Figur 19: eine perspektivische Darstellung einer flexiblen Hohlwelle gemäß einer achten Ausführungsform,
- Figur 20: eine Schnittdarstellung der flexiblen Hohlwelle nach Figur 19,
- Figur 21: eine perspektivische Darstellung einer flexiblen Hohlwelle gemäß einer
- Figur 22: neunten Ausführungsform, eine Schnittdarstellung der flexiblen Hohlwelle nach Figur 21,
- Figur 23: eine perspektivische Darstellung einer flexiblen Hohlwelle gemäß einer zehnten Ausführungsform,
- Figur 24: eine Schnittdarstellung der flexiblen Hohlwelle nach Figur 23,
- Figur 25: eine Seitenansicht der Hohlwelle nach Figur 23 in einem gebogenen Zustand,
- Figur 26: eine perspektivische Darstellung einer flexiblen Hohlwelle gemäß einer elften Ausführungsform,
- Figur 27: eine Schnittdarstellung der flexiblen Hohlwelle nach Figur 25,
- Figur 28: eine Seitenansicht des chirurgischen Instruments nach Figur 1 mit geöffnetem Maulteil,
- Figur 29: eine Seitenansicht eines chirurgischen Instruments mit einem Endeffektor der als Schere ausgeführt ist,
- Figur 30: eine Seitenansicht eines chirurgischen Instruments mit einem Endeffektor der als Pinzette ausgeführt ist.

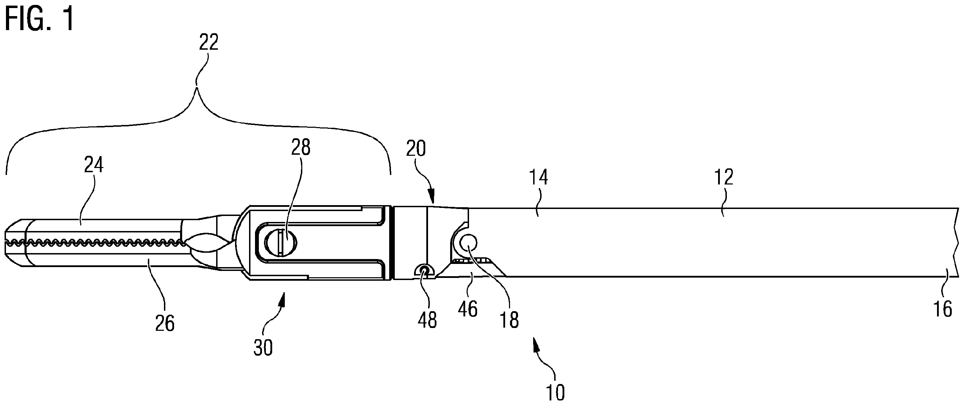

Figur 1 zeigt eine Seitenansicht eines chirurgisches Instruments 10 zur minimalinvasiven Chirurgie. Dieses Instrument 10 hat einen Instrumentenschaft 12 mit einem distalen Ende 14 und einem proximalen Ende 16. Der Instrumentenschaft 12 ist in Figur 1 zur besseren Darstellung des Instruments 10 verkürzt dargestellt. Das proximale Ende 16 des Instrumentenschafts 12 ist mit einem Manipulatorarm eines Manipulators verbindbar. Insbesondere umfasst das proximale Ende 16 des Instrumentenschafts 12 üblicherweise eine Instrumenteneinheit (nicht dargestellt), die über eine Schnittstelle mit dem Manipulatorarm verbindbar ist. Solche Manipulatoren werden auch als Telemanipulatorsysteme bezeichnet und dienen zur robotergestützten Chirurgie. Solche Manipulatoren sind insbesondere in den Dokumenten EP 3 025 667 A1 und WO 2016/083189 A1 offenbart. Das chirurgische Instrument 10 und die in den weiteren Figuren gezeigten Instrumente können mit einem entsprechenden Handgriff auch als laparoskopische Handinstrumente verwendet werden.

Das chirurgische Instrument 10 hat einen mit dem distalen Ende 14 des Instrumentenschafts 12 über ein als Scharnier 18 ausgebildetes Gelenk verbundenen Instrumentenkopf 20. In dem Instrumentenkopf 20 ist ein Endeffektor 22 um seine Längsachse drehbar gelagert. In Figur 1 ist der Endeffektor 22 als Greifer ausgebildet gezeigt und umfasst einen Endeffektorkörper 30, ein erstes Maulteil 24 und ein zweites Maulteil 26. Der Greifer ist in Figur 1 in einem geschlossenen Zustand dargestellt. Die Maulteile 24, 26 sind mit Hilfe eines Stifts 28 in dem Endeffektorkörper 30 drehbar gelagert und können um die Längsachse des Stifts 28 gedreht werden, um den Greifer zu öffnen und zu schließen. Weiterhin sind in Figur 1 ein Zughebel 46 und eine Schwenkachse 48 dargestellt.

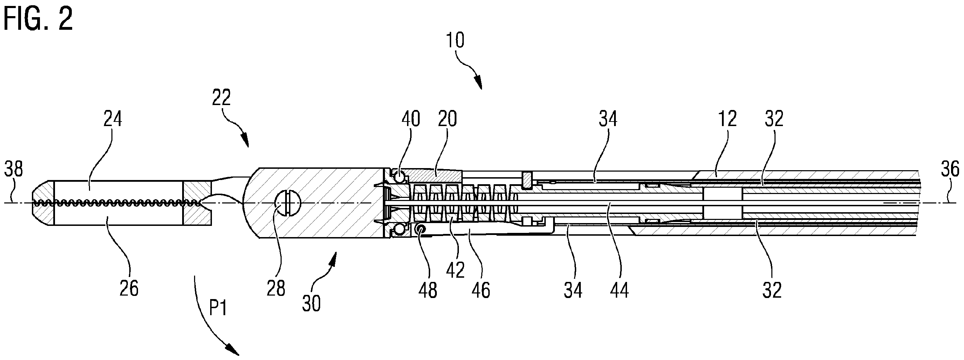

Figur 2 zeigt einen Längsschnitt des chirurgischen Instruments 10 nach Figur 1 entlang einer Mittelebene. Im Inneren des Instrumentenschafts 12 ist eine drehbar zum Instrumentenschaft 12 gelagerte Antriebswelle 32 sowie ein zwischen der Antriebswelle 32 und dem Instrumentenschaft 12 angeordneter Innenschaft 34 angeordnet, wobei der Innenschaft 34 zumindest in Längsrichtung des Instrumentenschafts 12 bewegt werden kann, um den Instrumentenkopf 20 um eine durch das Scharnier 18 (Figur 1) definierte Drehachse in Richtung des Pfeils P1 zu verschwenken. Der Pfeil P1 gibt somit die Schwenkrichtung des Instrumentenkopfs 20 an, in die er aus der in Figur 2 gezeigten Ruhelage herausbewegt werden kann. Zugleich wird der mit dem Instrumentenkopf 20 verbundene Endeffektor 22 aus der Ruhelage verschwenkt. Somit wird eine Längsachse 38 des Endeffektors 22 beziehungsweise des Instrumentenkopfs 20 zu einer Längsachse 36 des Instrumentenschafts 12 abgewinkelt. Der maximale Winkel, um den der Instrumentenkopf 20 in Richtung des Pfeils P1 verschwenkt beziehungsweise abgewinkelt werden kann, liegt bei 90°. Bei anderen Ausführungsformen können anstatt des Scharniers 18 auch andere Gelenke eingesetzt werden. Bei alternativen Ausführungsformen kann der maximale Winkel im Bereich von 60° bis 90° liegen, insbesondere bei 80°.

Der Instrumentenkopf 20 ist mit Hilfe eines Kugellagers 40 um die Längsachse 38 des Instrumentenkopfs 20 drehbar. Damit der Instrumentenkopf 20 auch bei einem verschwenkten Instrumentenkopf 20 gedreht werden kann, ist zur Übertragung der Drehbewegung eine flexible Hohlwelle 42 zwischen der Antriebswelle 32 und dem Instrumentenkopf 20 vorgesehen, deren proximales Ende mit der Antriebswelle 32 drehfest verbunden ist und deren distales Ende mit dem proximalen Ende des Endeffektorkörpers 30 drehfest verbunden ist. Die flexible Hohlwelle 42 ist im Inneren des Scharniers 18 angeordnet und überbrückt dieses.

Ein Verschwenken des Maulteils 24 um die durch den Stift 28 gebildete Drehachse wird durch einen Betätigungsdraht 44 gesteuert, wobei der Betätigungsdraht 44 zum Öffnen des Greifers eine Schubbewegung in Richtung des distalen Endes ausführt und zum Schließen des Greifers zum proximalen Ende hingezogen wird. Ein zweiter Betätigungsdraht kann vorgesehen sein, um das Maulteil 26, wie für das Maulteil 24 beschrieben, zu betätigen. Der Betätigungsdraht 44 verläuft im Inneren der flexiblen Hohlwelle 42 durch diese hindurch, so dass auch bei verschwenktem Instrumentenkopf 20 eine Betätigung des Greifers beziehungsweise allgemein eine Betätigung des Endeffektors 22 möglich ist. In alternativen Ausführungsformen ist nur ein Maulteil beweglich ausgestaltet.

Bei dem Endeffektor 22 oder bei anderen Endeffektoren kann über den Betätigungsdraht 44 zusätzlich eine elektrische Verbindung zum Endeffektor 22 hergestellt werden, beispielsweise um den Endeffektor 22 und das chirurgische Instrument 10 zur Hochfrequenzchirurgie einzusetzen. Alternativ oder zusätzlich können auch weitere Verbindungselemente, insbesondere elektrische und/oder mechanische Verbindungselemente, zum Endeffektor 22 hin vorgesehen sein und die insbesondere durch die flexible Hohlwelle 42 sowie durch die Antriebswelle 32, die ebenfalls als Hohlwelle ausgeführt ist, verlaufen. In einer weiteren alternativen Ausführungsform sind keine Verbindungselemente wie beispielsweise der Betätigungsdraht 44 zum Endeffektor vorgesehen, so dass die Antriebswelle massiv, das heißt nicht hohl, ausgeführt sein kann.

Der Innenschaft 34 steht mit dem proximalen Ende des Zughebels 46 in Verbindung. Der Zughebel 46 ist um die Schwenkachse 48 schwenkbar am Instrumentenkopf 20 angelenkt. Durch eine durch den Innenschaft 34 initiierte Bewegung des proximalen Endes des Zughebels 46 in Richtung des proximalen Endes des Instrumentenschafts 12 wird der Instrumentenkopf 20 um die Drehachse des Scharniers 18 stufenlos verschwenkt und die flexible Hohlwelle 42 entsprechend gebogen.

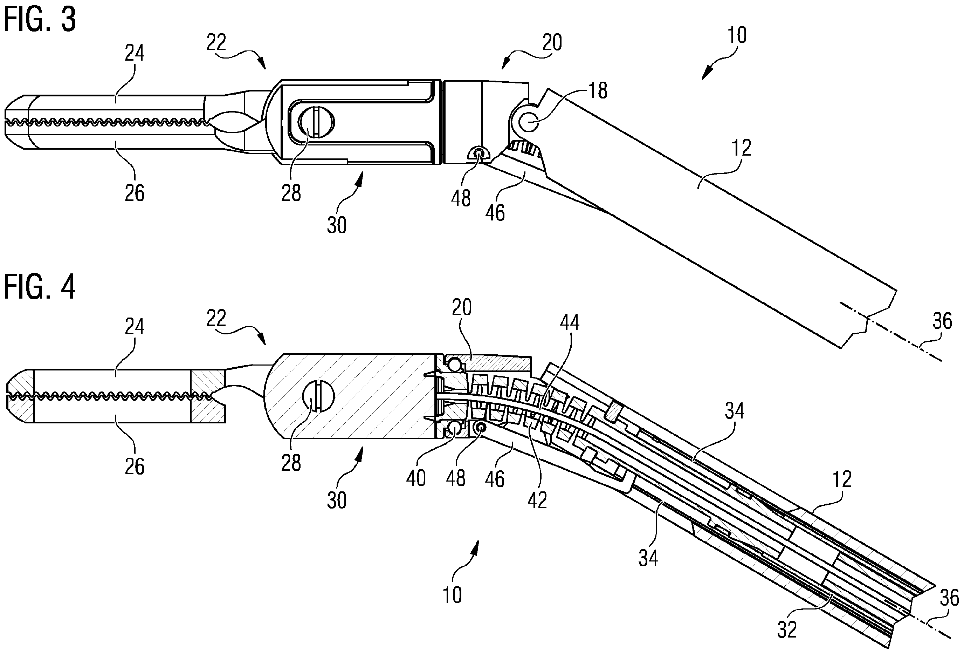

Figur 3 und 4 zeigen das chirurgische Instrument 10 nach Figur 1 und 2 mit einem abgewinkelten Instrumentenkopf 20 mit Endeffektor 22 in einer Seitenansicht beziehungsweise in einer Schnittzeichnung. Mit Hilfe der flexiblen Hohlwelle 42 ist auch in dem dargestellten abgewinkelten Zustand eine Übertragung eines Drehmoments von der Antriebswelle 32 zum Endeffektor 22 im Bereich des Gelenks 18 möglich. Die flexible Hohlwelle 42 ist torsionssteif und biegeelastisch ausgeführt. So kann ein Drehmoment übertragen werden, bei gleichzeitiger Abwinklung des Instrumentenkopfs 20 und Biegung der flexiblen Hohlwelle 42. Insbesondere liegt das maximal übertragbare Drehmoment in einem Bereich von 70 mNm bis 100 mNm. Eine Abwinklung des Instrumentenkopfs 20 mit dem Endeffektor 22 ist bis zu einem maximalen Winkel von 90° vorgesehen. Damit ist auch eine Abwinklung beziehungsweise Biegung der flexiblen Hohlwelle 42 um bis zu 90° vorgesehen.

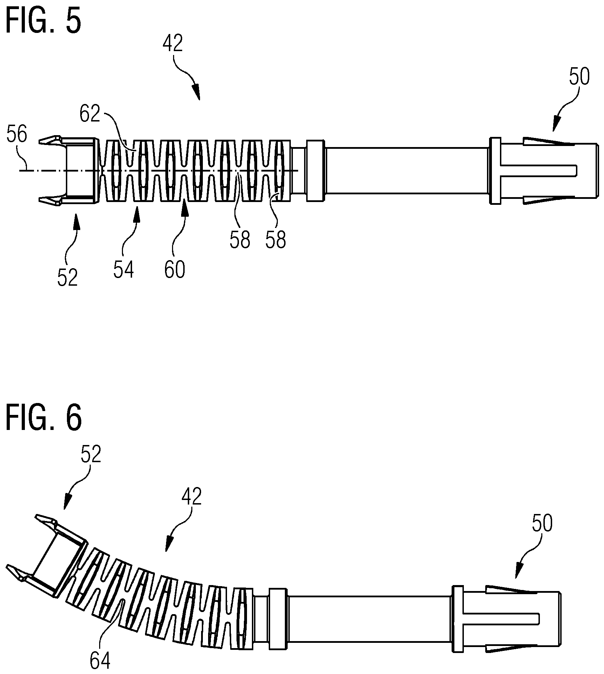

Figuren 5 und 6 zeigen eine Anordnung mit der Hohlwelle 42 in Detailansicht. Die flexible Hohlwelle 42 ist über einen starren Verbindungsbereich 50 mit einem distalen Ende der Antriebswelle 32 drehfest verbunden. Über einen gegenüberliegenden weiteren starren Verbindungsbereich 52 ist die flexible Hohlwelle 42 mit dem Endeffektor 22, insbesondere dem Endeffektorkörper 30, verbunden. So kann eine Drehung der Antriebswelle 32 auf den Endeffektor 22 übertragen werden. Auch in einem gebogenen Zustand der flexiblen Hohlwelle 42, wie er in Figur 6 gezeigt ist, ist eine kontinuierliche winkelsynchrone Übertragung einer Drehbewegung möglich. Insbesondere ist eine Endlosdrehung möglich, das heißt, eine Drehung um 360° und darüber hinaus.

Die flexible Hohlwelle 42 ist als Federstegkupplung ausgeführt. Im Folgenden werden die in Figur 5 dargestellten Merkmale der flexiblen Hohlwelle 42 detailliert beschrieben. Die flexible Hohlwelle 42 umfasst mehrere Federscheiben 54, die nebeneinander in jeweils einer Ebene orthogonal zu einer Längsachse 56 der Hohlwelle 42 angeordnet sind. In Längsrichtung entlang der Längsachse 56 der flexiblen Hohlwelle 42 benachbarte Federscheiben 54 sind jeweils durch zwei Verbindungsstege 58 miteinander verbunden. Bis auf die Verbindungsstege 58 sind die Federscheiben 54 durch Schlitze 60 in einer Wand der Hohlwelle 42 voneinander getrennt. Die Schlitze 60 erstrecken sich somit zwischen zwei Federscheiben 54 entlang eines Teils des Umfangs der flexiblen Hohlwelle 42. Die Schlitze 60 sind frei von Material beziehungsweise Werkstoffen, insbesondere frei von Polymeren, beispielsweise Silikon. Die Verbindungsstege 58, die zwei Federscheiben 54 verbinden, sind alle in einer Ebene orthogonal zur Längsachse 56 angeordnet.

Eine Federscheibe 54 der flexiblen Hohlwelle 42 umfasst mehrere Federstege 62, die jeweils zwischen zwei in Längsrichtung benachbarten einen Winkelversatz aufweisenden Verbindungsstegen 58 angeordnet sind und diese verbindet. Somit umfasst jede Federscheibe 54 vier Federstege 62, die im Wesentlichen entlang eines Kreisbogens am Umfang der Hohlwelle 42 angeordnet sind. Somit erstreckt sich eine Federscheibe 54 zusammen mit den entsprechenden Federstegen 62 im Wesentlichen in einer Ebene orthogonal zur Längsachse 56.

In Längsrichtung benachbarte Verbindungsstege 58 sind um die Längsachse 56 um 90° versetzt. Die zwei Verbindungsstege 58, die zwei nebeneinanderliegende Federscheiben 54 verbinden und somit in derselben Ebene orthogonal zur Längsachse 56 angeordnet sind, sind um 180° um die Längsachse 56 versetzt. Der Übergang 64 zwischen zwei in Längsrichtung benachbarten Federstegen 62 und dem diese Federstege 62 verbindenden Verbindungssteg 58 ist rund, insbesondere teilkreisförmig ausgeführt.

Alternativ können zwei nebeneinanderliegende Federscheiben 54 mit mehr als zwei Verbindungsstegen 58 verbunden sein, insbesondere mit drei oder vier Verbindungsstegen 58. Die Verbindungsstege 58 sind vorzugsweise voneinander gleichmäßig beabstandet, so dass sich bei drei Verbindungsstegen 58 ein Winkel um die Längsachse 56 zwischen benachbarten Verbindungsstegen 58 von 120°, bei vier Verbindungsstegen 58 ein Winkel zwischen benachbarten Verbindungsstegen 58 von 90° ergibt.

Die Federscheiben 54 der flexiblen Hohlwelle 42 sind im gestreckten beziehungsweise geraden Zustand (siehe Figur 5), in dem die Hohlwelle 42 nicht gebogen ist, entlang der Längsachse 56 mit gleichem Abstand zueinander angeordnet. Im gebogenen Zustand der flexiblen Hohlwelle 42 (siehe Figur 6) verringert sich die Breite der Schlitze 60 beziehungsweise der Abstand zwischen nebeneinanderliegenden Federscheiben 54 an der bezüglich der Biegerichtung inneren Hohlwellenseite. Auf einer gegenüberliegenden äußeren Seite der Hohlwelle 42 vergrößert sich der Abstand zwischen nebeneinanderliegenden Federscheiben 54. Dabei ermöglichen die Federstege 62 als elastische Elemente eine elastische Verformung der Federscheiben 54 zueinander.

Weiterhin ist die flexible Hohlwelle 42 so ausgebildet, dass bei maximaler Biegung der flexiblen Hohlwelle 42, entsprechend einer maximalen Verschwenkungsposition des Instrumentenkopfs 20, die Federscheiben 54 an der bezüglich der Biegerichtung inneren Hohlwellenseite gerade in Anlage kommen oder noch voneinander beabstandet sind. Die maximale Biegung der flexiblen Hohlwelle 42 ist bei 90° erreicht. Auch bei maximaler Biegung der Hohlwelle 42 ist eine Drehung der Hohlwelle mit Hilfe der Antriebswelle 32 möglich.

Das chirurgische Instrumente 10 hat einen maximalen Außendurchmesser von 5 mm. Weiterhin hat die im chirurgischen Instrument 10 verbaute flexible Hohlwelle 42 einen Außendurchmesser von 2,8 mm und einen Innendurchmesser von 2,1 mm, die Wanddicke entspricht somit 0,7 mm. Das Verhältnis von Außendurchmesser zu Innendurchmesser ist somit 1,33. Die Länge der flexiblen Hohlwelle 42 ist 8,45 mm. Weiterhin umfasst die Hohlwelle 42 dreizehn nebeneinanderliegende beziehungsweise benachbarte Federscheiben 54.

Selbstverständlich kann der maximale Außendurchmesser des chirurgischen Instruments 10 ein alternatives Maß haben. Weiterhin kann alternativ der Außendurchmesser, der Innendurchmesser und/oder die Länge derflexiblen Hohlwelle 42 von den genannten Maßen abweichen.

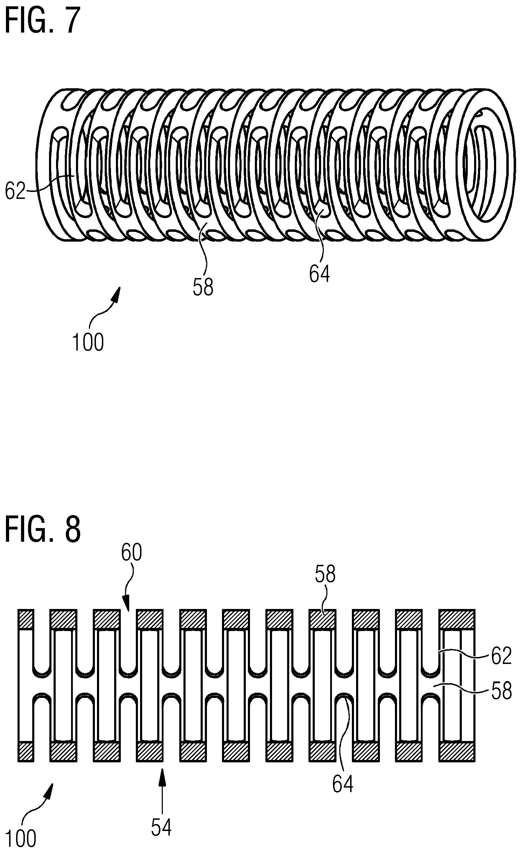

In den folgenden Figuren 7 bis 26 sind alternative Ausführungsformen der flexiblen Hohlwelle 42 im Detail dargestellt. Elemente mit gleichem Aufbau oder gleicher Funktion haben dieselben Bezugszeichen. Diese alternativen Ausführungsformen können anstatt der flexiblen Hohlwelle 42 in dem chirurgischen Instrument 10 verbaut sein. Weiterhin können die alternativen Ausführungsformen mit Verbindungsbereichen 50, 52 verbunden sein, so dass die alternativen Ausführungsformen die flexible Hohlwelle 42 in der in Figuren 5 und 6 gezeigten Anordnung ersetzten.

Figur 7 zeigt eine perspektivische Darstellung einer flexiblen Hohlwelle 100. Figur 8 zeigt eine Schnittdarstellung der flexiblen Hohlwelle 100, die in einer Ebene entlang der Längsachse der flexiblen Hohlwelle 100 geschnitten ist. Die flexible Hohlwelle 100 umfasst parallele Federscheiben 54 mit parallelen Federstegen 62 und je zwei Verbindungsstege 58, die zwei benachbarte Federscheiben 54 verbinden. Die Federscheiben 54 und Federstege 62 sind insbesondere dann parallel, wenn die flexible Hohlwelle 100 in einem entspannten Zustand ist, wie er in Figuren 7 und 8 dargestellt ist. Der Übergang 64 zwischen Federstegen 62 und dem Verbindungssteg 58 ist bei der Hohlwelle 100 rund, insbesondere kreisrund, ausgeführt. Die zwei Verbindungsstege 58, die zwei Federscheiben 54 verbinden, sind um 180° um die Längsachse der Hohlwelle 100 versetzt. Die in Längsrichtung benachbarten Verbindungsstege 58 sind um die Längsachse der Hohlwelle 100 um 90° zueinander versetzt. Weiterhin hat das Verhältnis von Breite eines Schlitzes 60 zur Breite eines Federstegs 62 der Hohlwelle 100 einen Wert von 4, wobei die Hohlwelle 100 neunzehn Federscheiben 54 umfasst. Bei anderen Ausführungsformen kann das Verhältnis einen Wert im Bereich von 2 bis 7 haben.

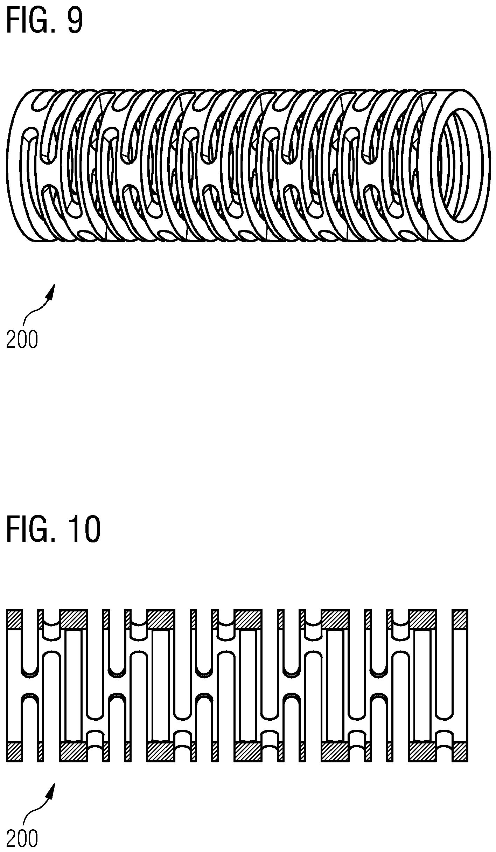

Figur 9 zeigt eine perspektivische Darstellung einer flexiblen Hohlwelle 200. Figur 10 zeigt eine Schnittdarstellung der flexiblen Hohlwelle 200, die in einer Ebene entlang der Längsachse der flexiblen Hohlwelle 200 geschnitten ist. Die Verbindungsstege 58, die zwei Federscheiben 54 verbinden, sind um 180° um die Längsachse der Hohlwelle 200 versetzt. Die in Längsrichtung benachbarten Verbindungsstege 58 sind um die Längsachse der Hohlwelle 200 um 45° versetzt. Somit sind die zwei Verbindungsstege 58, die in einer ersten Ebene orthogonal zur Längsachse der Hohlwelle 200 angeordnet sind, jeweils zu den zwei Verbindungsstegen 58 einer benachbarten zweiten Ebene um 45° um die Längsachse der Hohlwelle 200 versetzt. Die Verbindungsstege 58 beschreiben somit in Längsrichtung und um den Umfang der Hohlwelle 200 eine Helix-Form im oder gegen den Uhrzeigersinn. Das Verhältnis von Breite eines Schlitzes 60 zur Breite eines Federstegs 62 der Hohlwelle 200 hat einen Wert von 3. Bei anderen Ausführungsformen kann das Verhältnis einen Wert im Bereich von 2 bis 7 haben.

Alternativ zu der Anordnung der Verbindungsstege 58 der Hohlwelle 200, können die Verbindungsstege 58 der benachbarten Ebenen jeweils um einen Winkel im Bereich von 20° bis 89°, insbesondere in einem Bereich von 45° bis 89°, um die Längsachse der Hohlwelle 200 versetzt sein.

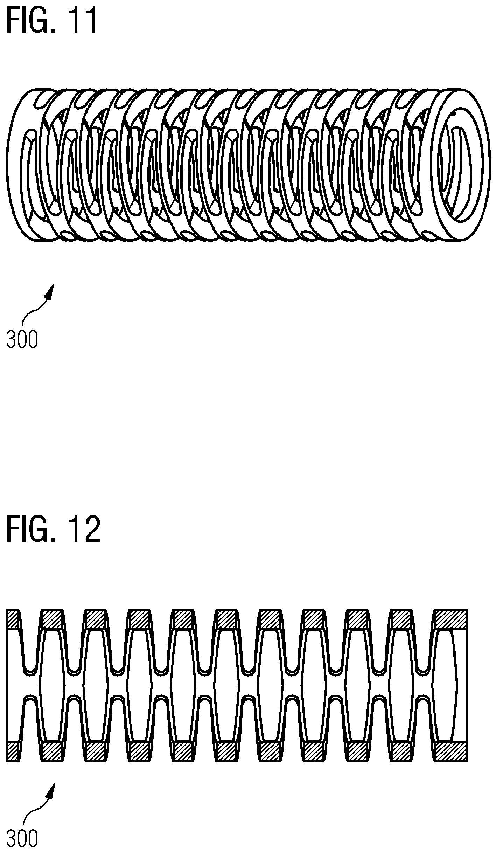

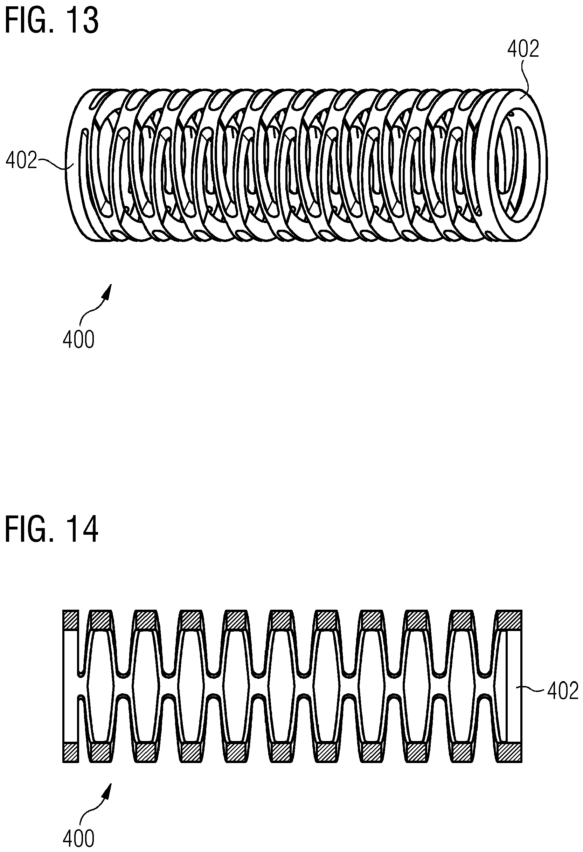

Figur 11 zeigt eine perspektivische Darstellung einer flexiblen Hohlwelle 300. Figur 12 zeigt eine Schnittdarstellung der flexiblen Hohlwelle 300, die in einer Ebene entlang der Längsachse der flexiblen Hohlwelle 300 geschnitten ist. Die flexible Hohlwelle 300 umfasst Federstege 62, die X-förmig angeordnet sind, so dass jeweils vier Federstege 62, die an einen Verbindungsstege 58 angrenzen, zusammen mit dem einen Verbindungssteg 58 eine X-Form bilden. Dabei ist der eine Verbindungssteg im Zentrum der X-Form. Ausgehend von diesem Verbindungssteg 58 gehen die vier Federstege 62 so weg, dass sich der Abstand zwischen zwei Federstegen 62 mit Abstand von dem Verbindungssteg 58 vergrößert. Das Verhältnis von Breite eines Schlitzes 60 zur Breite eines Federstegs 62 der Hohlwelle 300 hat einen Wert von 5,625. Dazu wird die Breite eines Schlitzes 60 in Längsrichtung zwischen zwei Verbindungsstegen 58 gemessen. Die Breite eines Federstegs 62 ist gleichbleibend über seine Länge zwischen zwei Verbindungsstegen 58. Die Breite eines Federstegs 62 wird mittig zwischen zwei in Längsrichtung benachbarten Verbindungsstegen 58 gemessen. Bei anderen Ausführungsformen kann das Verhältnis einen Wert im Bereich von 2 bis 7 haben. Figur 13 zeigt eine perspektivische Darstellung einer flexiblen Hohlwelle 400. Figur 14 zeigt eine Schnittdarstellung der flexiblen Hohlwelle 400, die in einer Ebene entlang der Längsachse der flexiblen Hohlwelle 400 geschnitten ist. Die Hohlwelle 400 umfasst verkürzte Verbindungsstege 58, die jeweils die äußersten Federscheiben 54 der Hohlwelle 400 mit einem ringförmigen Endstück 402 verbinden. Das Verhältnis von Breite eines Schlitzes 60 zur Breite eines Federstegs 62 der Hohlwelle 400 hat einen Wert von 6. Im Vergleich zur Hohlwelle 300 ist somit bei gleichbleibender Länge der Hohlwellen und gleichbleibender Breite der Federstege 62 der Abstand zwischen benachbarten Federscheiben 54 der Hohlwelle 400 erhöht. Dazu wird die Breite eines Schlitzes 60 in Längsrichtung zwischen zwei Verbindungsstegen 58 gemessen. Die Breite eines Federstegs 62 ist gleichbleibend über seine Länge zwischen zwei Verbindungsstegen 58. Bei anderen Ausführungsformen kann das Verhältnis einen Wert im Bereich von 2 bis 7 haben.

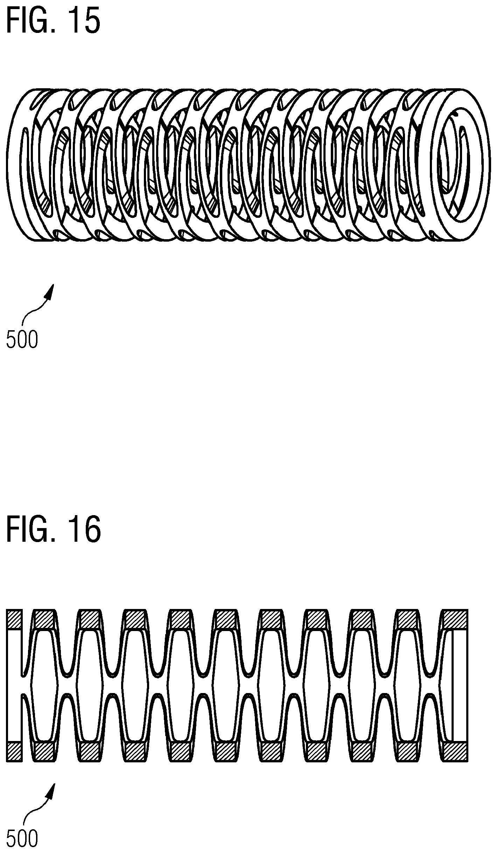

Figur 15 zeigt eine perspektivische Darstellung einer flexiblen Hohlwelle 500. Figur 16 zeigt eine Schnittdarstellung der flexiblen Hohlwelle 500, die in einer Ebene entlang der Längsachse der flexiblen Hohlwelle 500 geschnitten ist. Die Hohlwelle 500 umfasst, wie für Figur 13 beschrieben, verkürzte Verbindungsstege 58. Die Hohlwelle 500 hat neben X-förmig angeordneten Federstegen 62 einen Übergang 64 zwischen zwei benachbarten Federstegen 62 der parabelförmig ist. Die Parabelform ist dabei so angeordnet, dass der Scheitelpunkt der Parabel an einem Verbindungssteg 58 anliegt und die Äste der Parabel jeweils einen Federsteg 62 zum Schlitz 60 hin begrenzen. Das Verhältnis von Breite eines Schlitzes 60 zur Breite eines Federstegs 62 der Hohlwelle 500 hat einen Wert von 6. Dazu wird die Breite eines Schlitzes 60 in Längsrichtung zwischen zwei Verbindungsstegen 58 gemessen. Die Breite eines Federstegs 62 wird mittig zwischen zwei in Längsrichtung benachbarten Verbindungsstegen 58 gemessen. An diesem Punkt haben die Federstege 62 ihre geringste Breite. Bei anderen Ausführungsformen kann das Verhältnis einen Wert im Bereich von 2 bis 7 haben.

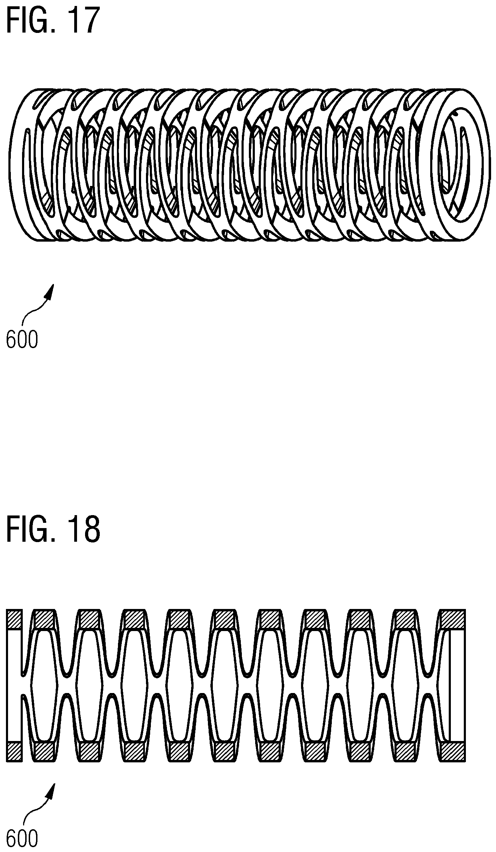

Figur 17 zeigt eine perspektivische Darstellung einer flexiblen Hohlwelle 600. Figur 18 zeigt eine Schnittdarstellung der flexiblen Hohlwelle 600, die in einer Ebene entlang der Längsachse der flexiblen Hohlwelle 600 geschnitten ist. Die Hohlwelle 600 umfasst, wie für Figur 13 beschrieben, verkürzte Verbindungsstege 58. Die Hohlwelle 600 hat neben X-förmig angeordneten Federstegen 62 ebenfalls einen parabelförmigen Übergang 64. Das Verhältnis von Breite eines Schlitzes 60 zur Breite eines Federstegs 62 der Hohlwelle 600 hat einen Wert von 4,8. Dazu wird die Breite eines Schlitzes 60 in Längsrichtung zwischen zwei Verbindungsstegen 58 gemessen. Die Breite eines Federstegs 62 wird mittig zwischen zwei in Längsrichtung benachbarten Verbindungsstegen 58 gemessen. An diesem Punkt haben die Federstege 62 ihre geringste Breite. Bei anderen Ausführungsformen kann das Verhältnis einen Wert im Bereich von 2 bis 7 haben.

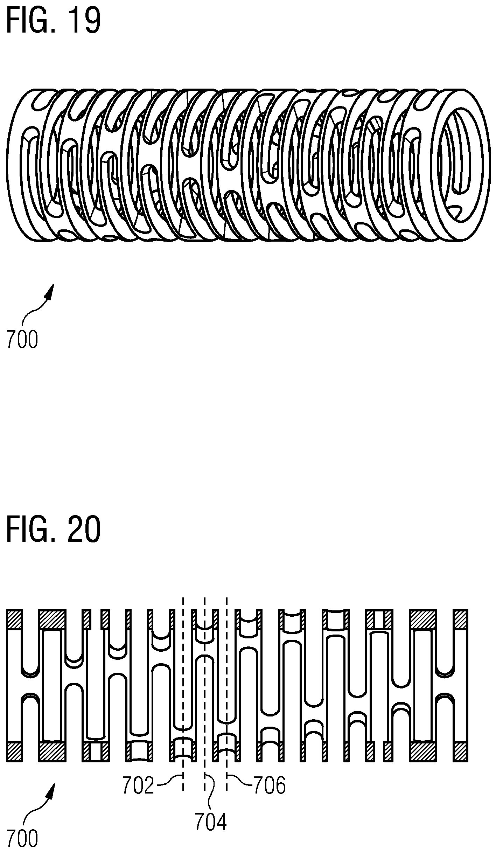

Figur 19 zeigt eine perspektivische Darstellung einer flexiblen Hohlwelle 700. Figur 20 zeigt eine Schnittdarstellung der flexiblen Hohlwelle 700, die in einer Ebene entlang der Längsachse der flexiblen Hohlwelle 700 geschnitten ist. Die Verbindungsstege 58 sind um den Umfang der Hohlwelle 700 im oder gegen den Uhrzeigersinn so angeordnet, dass sie in Längsrichtung der Hohlwelle 700 einer Helix-Form folgen. Die zwei Verbindungsstege 58, die in einer ersten Ebene 702 orthogonal zur Längsachse der Hohlwelle 700 angeordnet sind, sind jeweils zu den zwei Verbindungsstegen 58 einer übernächsten dritten Ebene 706 um 10° um die Längsachse der Hohlwelle 700 versetzt. Die zwei Verbindungsstege 58, die in einer zur ersten Ebene 702 benachbarten zweiten Ebene 704 angeordnet sind, sind jeweils um 90° zu den Verbindungsstegen 58 der ersten Ebene 702 versetzt. Das Verhältnis von Breite eines Schlitzes 60 zur Breite eines Federstegs 62 der Hohlwelle 700 hat einen Wert von 4. Dazu wird die Breite eines Schlitzes 60 in Längsrichtung zwischen zwei Verbindungsstegen 58 gemessen. Die Breite eines Federstegs 62 ist gleichbleibend über seine Länge zwischen zwei Verbindungsstegen 58. Bei anderen Ausführungsformen kann das Verhältnis einen Wert im Bereich von 2 bis 7 haben.

Alternativ können die zwei Verbindungsstege 58 einer zweiten Ebene 704 jeweils zu den zwei Verbindungsstegen 58 einer ersten Ebene 702 um 95° versetzt sein und die zwei Verbindungsstege 58 einer dritten Ebene 706 jeweils um 95° zu den zwei Verbindungsstegen 58 der zweiten Ebene 704 versetzt sein. Somit sind die zwei Verbindungsstege 58 der dritten Ebene 706 jeweils um 10° zu den Verbindungsstegen 58 der ersten Ebene 702 um die Längsachse versetzt.

Weiterhin kann alternativ der Versatzwinkel zwischen den Verbindungsstegen 58 der ersten Ebene 702 und der dritten Ebene 706 jeweils einen Wert im Bereich von 2° bis 40° um die Längsachse der Hohlwelle 700 versetzt sein.

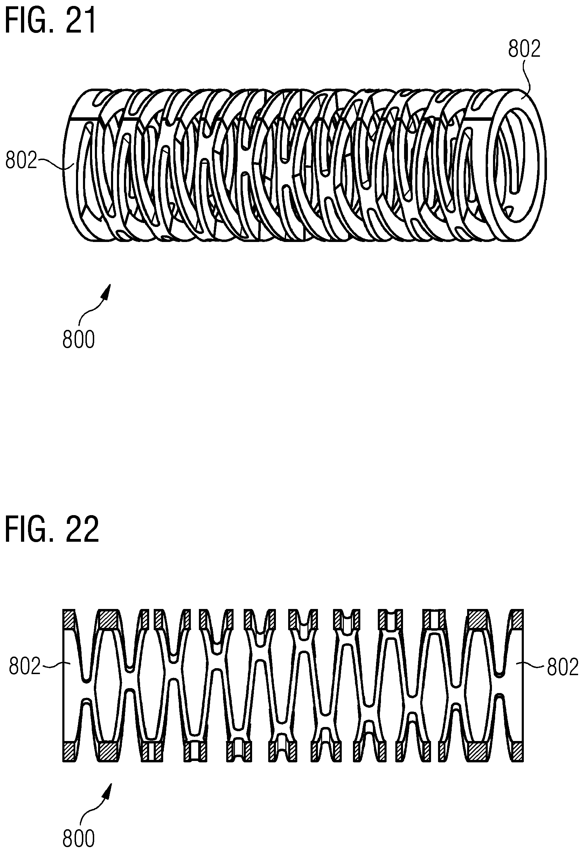

Figur 21 zeigt eine perspektivische Darstellung einer flexiblen Hohlwelle 800. Figur 22 zeigt eine Schnittdarstellung der flexiblen Hohlwelle 800, die in einer Ebene entlang der Längsachse der flexiblen Hohlwelle 800 geschnitten ist. Die Hohlwelle 800 hat neben X-förmig angeordneten Federstegen 62 helixförmig angeordnete Verbindungsstege 58. Weiterhin beschreiben auch die Endstücke 802 der Hohlwelle 800, insbesondere deren Seiten zu einem Schlitz, mit der jeweils benachbarten Federscheibe 54 eine X-Form. Das Verhältnis von Breite eines Schlitzes 60 zur Breite eines Federstegs 62 der Hohlwelle 700 hat einen Wert von 3,875 oder 4,65, wobei in Längsrichtung nebeneinanderliegende Federstege 62 jeweils unterschiedlich breit sind. Die Breite eines Schlitzes 60 in Längsrichtung wird zwischen zwei Verbindungsstegen 58 gemessen. Die Breite eines Federstegs 62 ist gleichbleibend über seine Länge zwischen zwei Verbindungsstegen 58. Die unterschiedlichen Breiten der Federstege 62 tragen zur Stabilität der X-förmig angeordneten Federstege 62 der flexiblen Hohlwelle 800 bei, insbesondere in Zusammenhang mit den helixförmig angeordneten Verbindungsstegen 58. Bei anderen Ausführungsformen kann das Verhältnis einen Wert im Bereich von 2 bis 7 haben.

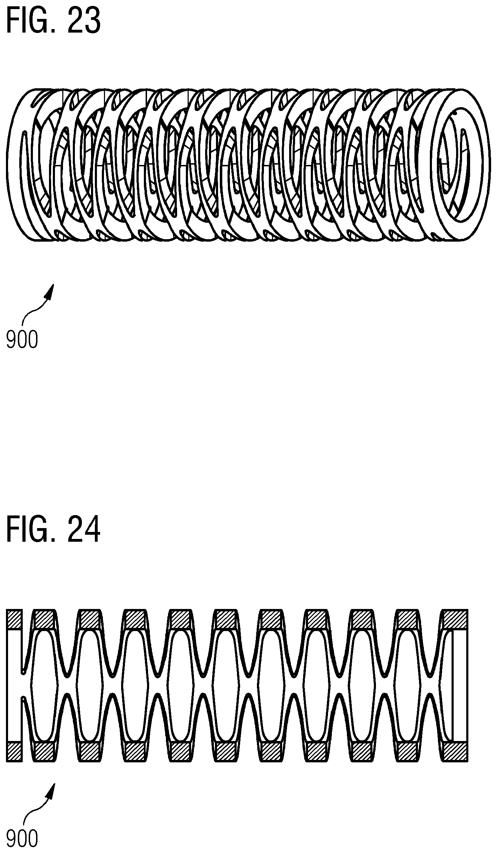

Figur 23 zeigt eine perspektivische Darstellung einer flexiblen Hohlwelle 900. Figur 24 zeigt eine Schnittdarstellung der flexiblen Hohlwelle 900, die in einer Ebene entlang der Längsachse der flexiblen Hohlwelle 900 geschnitten ist. Die Hohlwelle 900 hat neben X-förmig angeordneten Federstegen 62 auch parabelförmige Übergänge 64. Weiterhin sind die Federstege 62 der Hohlwelle 900 so ausgebildet, dass sich der Querschnitt eines Federstegs 62 ausgehend von der Mitte eines Federstegs 62 zwischen zwei Verbindungsstegen 58 zu den jeden der Verbindungsstege 58 hin vergrößert. Der Querschnitt eines Federstegs 62 ist dabei das Produkt aus Breite und Tiefe des Federstegs 62, wobei die Tiefe des Federstegs 62 in radialer Richtung ausgehend von der Längsachse der Hohlwelle 900 konstant ist. Somit verändert sich die Breite des Federstegs 62 in Längsrichtung der Hohlwelle 900. Das Verhältnis von Breite eines Schlitzes 60 zur Breite eines Federstegs 62 der Hohlwelle 900 hat einen Wert von 6. Dazu wird die Breite eines Schlitzes 60 in Längsrichtung zwischen zwei Verbindungsstegen 58 gemessen. Die Breite eines Federstegs 62 wird mittig zwischen zwei in Längsrichtung benachbarten Verbindungsstegen 58 gemessen. An diesem Punkt haben die Federstege 62 ihre geringste Breite. Bei anderen Ausführungsformen kann das Verhältnis einen Wert im Bereich von 2 bis 7 haben.

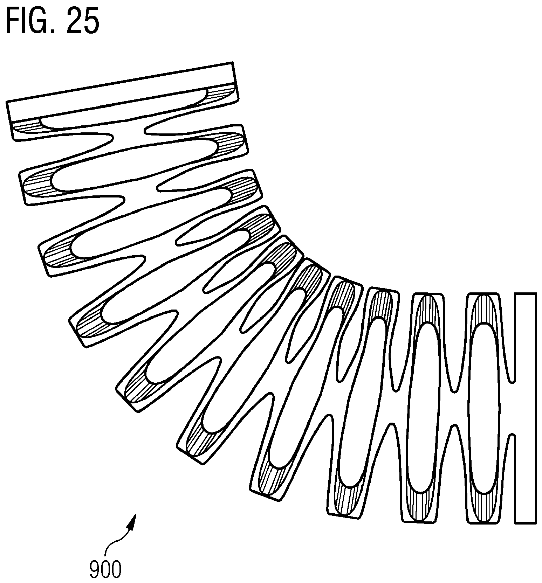

Figur 25 zeigt eine Seitenansicht der Hohlwelle 900 nach Figur 23 in einem gebogenen Zustand. Der Biegewinkel entspricht 80°. Die Federstege 62 der flexiblen Hohlwelle 900 verformen sich elastisch bei Biegung der Hohlwelle 900 und erlauben eine Drehung der Hohlwelle 900. Die Federscheiben 54 der Hohlwelle 900 berühren sich dabei nicht.

Die Hohlwelle 900 kann über 80° hinaus gebogen werden, insbesondere bis 90°. Bei Biegung bis 90° kommen die Federscheiben 54 der Hohlwelle 900 in Berührung, insbesondere die Federscheiben 54 in der Mitte der Hohlwelle 900. Außerdem verringert sich der Abstand der restlichen Federscheiben 54 zueinander an der bezüglich der Biegerichtung inneren Hohlwellenseite.

Weiterhin ist es alternativ möglich, eine verlängerte flexible Hohlwelle 42, 100, 200, 300, 400, 500, 600, 700, 800, 900, 1000 einzusetzen, um so eine größere maximale Biegung zu ermöglichen, insbesondere bis zu 120°. Die verlängerte Hohlwelle 42, 100, 200, 300, 400, 500, 600, 700, 800, 900, 1000 kann eine größere Anzahl an Federscheiben 54 umfassen. Jede Federscheibe 54 kann dabei beispielsweise nach einem für die Ausführungsformen der flexiblen Hohlwelle 42, 100, 200, 300, 400, 500, 600, 700, 800, 900, 1000 ausgebildet sein.

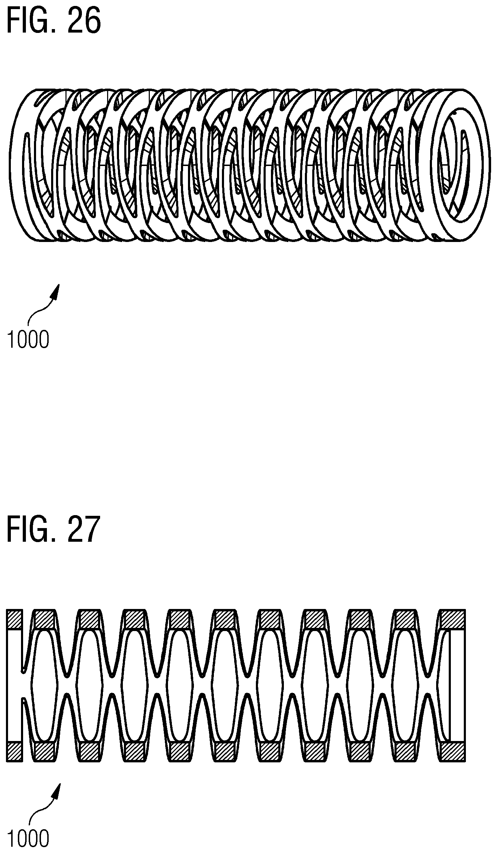

Figur 26 zeigt eine perspektivische Darstellung einer flexiblen Hohlwelle 1000. Figur 27 zeigt eine Schnittdarstellung der flexiblen Hohlwelle 1000, die in einer Ebene entlang der Längsachse der flexiblen Hohlwelle 1000 geschnitten ist. Die Hohlwelle 1000 hat, wie für die Hohlwelle 900 beschrieben, Federstege 62 mit einem sich vergrößernden Querschnitt. Abweichend hat das Verhältnis von Breite eines Schlitzes 60 zur Breite eines Federstegs 62 der Hohlwelle 1000 einen Wert von 4,8. Dazu wird die Breite eines Schlitzes 60 in Längsrichtung zwischen zwei Verbindungsstegen 58 gemessen. Die Breite eines Federstegs 62 wird mittig zwischen zwei in Längsrichtung benachbarten Verbindungsstegen 58 gemessen. An diesem Punkt haben die Federstege 62 ihre geringste Breite. Bei anderen Ausführungsformen kann das Verhältnis einen Wert im Bereich von 2 bis 7 haben.

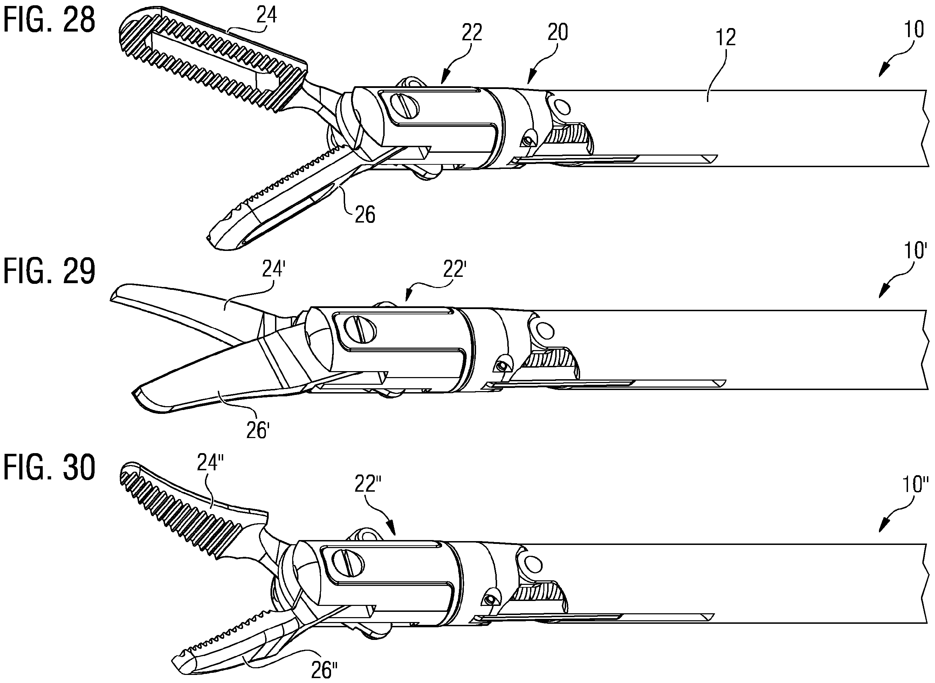

Figur 28 zeigt eine Seitenansicht des chirurgische Instrument 10 mit geöffnetem Maulteil.

Figuren 29 und 30 zeigen chirurgische Instrumente 10', 10" mit alternativen Endeffektoren 22', 22". Figur 29 zeigt eine Seitenansicht eines chirurgischen Instruments 10' mit einem Endeffektor 22' der als Schere ausgebildet ist. Die Scherenklingen 24', 26' sind dabei wie die Maulteile 24, 26 im Endeffektorkörper gelagert und können geöffnet und geschlossen werden um insbesondere Gewebe zu schneiden.

Figur 30 zeigt eine Seitenansicht eines chirurgischen Instrument 10" mit einem Endeffektor 22" der als Dissektor ausgeführt ist. Die Dissektor-Schenkel 24", 26" sind dabei wie die Maulteile 24, 26 im Endeffektorkörper 30 gelagert und können geöffnet und geschlossen werden um insbesondere Gewebe zu fassen oder aufzuspreizen.

Weiterhin kann der Endeffektor 22, 22', 22" alternativ als Klemme, als Heftvorrichtung, als Haken, oder als ein anderes chirurgisches Werkzeug ausgebildet sein.

### Bezugszeichenliste

- 10, 10', 10": Chirurgisches Instrument
- 12: Instrumentenschaft
- 14: Distales Ende des Instrumentenschafts
- 16: Proximales Ende des Instrumentenschafts
- 18: Scharnier
- 20: Instrumentenkopf
- 22: Endeffektor
- 24,26: Maulteile
- 24', 26': Scherenklingen
- 24", 26": Dissektor-Schenkel
- 28: Stift
- 30: Endeffektorkörper
- 32: Antriebswelle
- 34: Innenschaft
- 36: Längsachse des Instrumentenschafts
- 38: Längsachse des Endeffektors
- 40: Kugellager
- 42, 100, 200, 300, 400, 500,:
- 600, 700, 800, 900, 1000: flexible Hohlwelle
- 44: Betätigungsdraht
- 46: Zughebel
- 48: Schwenkachse
- 50,52: Starrer Verbindungsbereich
- 54: Federscheibe
- 56: Längsachse der flexiblen Hohlwelle
- 58: Verbindungssteg
- 60: Schlitz
- 62: Federsteg
- 64: Übergang
- 402,802: Endstück
- 702: Erste Ebene
- 704: Zweite Ebene
- 706: Dritte Ebene

## Patentansprüche

1. Chirurgisches Instrument zur minimalinvasiven Chirurgie,
mit einem Instrumentenschaft (12) mit einem distalen Ende (14),
mit einem Instrumentenkopf (20), der über mindestens ein Gelenk schwenkbar mit dem distalen Ende des Instrumentenschafts (12) verbunden ist,
mit einem Endeffektor (22), der um seine Längsachse (38) drehbar im Instrumentenkopf (20) gelagert ist,
mit einer zum Instrumentenschaft (12) koaxialen Antriebswelle (32) zur Drehung des Endeffektors (22),
wobei das chirurgische Instrument (10, 10', 10") an einem proximalen Ende mit einer manuellen Betätigungseinrichtung oder mit einem Robotersystem zur Betätigung zumindest des Instrumentenschafts (12) und der Antriebswelle (32) verbindbar ist,
mit einer flexiblen Hohlwelle (42, 100, 200, 300, 400, 500, 600, 700, 800, 900, 1000), die eine Drehbewegung von einem Ende der Antriebswelle (32) zum Endeffektor (22) zumindest im Bereich des Gelenks überträgt,
wobei die flexible Hohlwelle (42, 100, 200, 300, 400, 500, 600, 700, 800, 900, 1000) mehrere in Längsrichtung der Hohlwelle (42, 100, 200, 300, 400, 500, 600, 700, 800, 900, 1000) nebeneinander angeordnete Federscheiben (54) umfasst, die durch in der Wandung der Hohlwelle (42, 100, 200, 300, 400, 500, 600, 700, 800, 900, 1000) radial erstreckende Schlitze (60) entlang eines Teils des Umfangs der Hohlwelle (42, 100, 200, 300, 400, 500, 600, 700, 800, 900, 1000) voneinander getrennt sind,
wobei in Längsrichtung der Hohlwelle (42, 100, 200, 300, 400, 500, 600, 700, 800, 900, 1000) benachbarte Federscheiben (54) durch mindestens zwei Verbindungstege (58) miteinander verbunden sind, und
wobei in Längsrichtung der Hohlwelle (42, 100, 200, 300, 400, 500, 600, 700, 800, 900, 1000) benachbarte Verbindungsstege (58) jeweils durch Federstege (62) einer Federscheibe (54) verbunden sind.

2. Chirurgisches Instrument nach Anspruch 1, wobei die Federscheiben (54) in einem nicht abgewinkelten Zustand des Instrumentenkopfs (20) jeweils in einer orthogonal zur Längsachse (56) der Hohlwelle verlaufenden Ebene parallel zueinander angeordnet sind.

3. Chirurgisches Instrument nach einem der vorhergehenden Ansprüche, wobei in einem nicht abgewinkelten Zustand des Instrumentenkopfs (20) die vier Federstege (62), die an einen Verbindungssteg (58) angrenzen, in parallelen Ebenen verlaufen, oder
wobei in einem nicht abgewinkelten Zustand des Instrumentenkopfs (20) die vier Federstege (62), die an einen Verbindungssteg (58) angrenzen, zusammen mit dem Verbindungssteg (58) eine X-Form bilden.

4. Chirurgisches Instrument nach einem der vorhergehenden Ansprüche, wobei die Verbindungsstege (58), die zwei nebeneinanderliegende Federscheiben (54) verbinden, entlang eines Kreisbogens am Umfang der Hohlwelle (42, 100, 200, 300, 400, 500, 600, 700, 800, 900, 1000) gleichmäßig voneinander beabstandet sind.

5. Chirurgisches Instrument nach einem der vorhergehenden Ansprüche, wobei die Verbindungsstege (58) einer ersten Ebene orthogonal zur Längsachse (56) der Hohlwelle (42, 100, 200, 300, 400, 500, 600, 700, 800, 900, 1000) jeweils zu Verbindungsstegen (58) einer benachbarten zweiten Ebene um 90° um die Längsachse (56) der Hohlwelle (42, 100, 200, 300, 400, 500, 600, 700, 800, 900, 1000) versetzt sind.

6. Chirurgisches Instrument nach einem der vorhergehenden Ansprüche 1 bis 4, wobei die Verbindungsstege (58) um die Längsachse (56) der Hohlwelle (42, 100, 200, 300, 400, 500, 600, 700, 800, 900, 1000) so zueinander versetzt angeordnet sind, dass sie in Längsrichtung der Hohlwelle (42, 100, 200, 300, 400, 500, 600, 700, 800, 900, 1000) einer Helix-Form folgen.

7. Chirurgisches Instrument nach Anspruch 6, wobei die Verbindungsstege (58) einer ersten Ebene jeweils zu Verbindungsstegen (58) einer benachbarten zweiten Ebene um 20° bis 89° oder um 91° bis 110° um die Längsachse (56) der Hohlwelle (42, 100, 200, 300, 400, 500, 600, 700, 800, 900, 1000) versetzt sind, oder wobei die Verbindungstege (58) einer ersten Ebene jeweils zu Verbindungsstegen (58) einer benachbarten zweiten Ebene um 90° um die Längsachse (56) der Hohlwelle (42, 100, 200, 300, 400, 500, 600, 700, 800, 900, 1000) versetzt sind und jeweils zu Verbindungsstegen (58) einer übernächsten dritten Ebene um 2° bis 40° um die Längsachse (56) der Hohlwelle (42, 100, 200, 300, 400, 500, 600, 700, 800, 900, 1000) versetzt sind.

8. Chirurgisches Instrument nach einem der vorhergehenden Ansprüche, wobei sich der Querschnitt eines Federstegs (62) ausgehend von der Mitte eines Federstegs (62) zwischen zwei Verbindungsstegen (58) zu den jeden der Verbindungsstege (58) hin vergrößert.

9. Chirurgisches Instrument nach einem der vorhergehenden Ansprüche, wobei der Übergang (64) zwischen zwei in Längsrichtung der Hohlwelle (42, 100, 200, 300, 400, 500, 600, 700, 800, 900, 1000) benachbarten Federstegen (62) im Bereich eines Verbindungsstegs (58) teilkreisförmig oder parabelförmig ist.

10. Chirurgisches Instrument nach einem der vorhergehenden Ansprüche, wobei die flexible Hohlwelle (42, 100, 200, 300, 400, 500, 600, 700, 800, 900, 1000) bei Rotation um die Längsachse (56) der Hohlwelle (42, 100, 200, 300, 400, 500, 600, 700, 800, 900, 1000) torsionssteif ist und gleichzeitig biegeelastisch bei Abwinklung des Instrumentenkopf (20) ist.

11. Chirurgisches Instrument nach einem der vorhergehenden Ansprüche, wobei der Instrumentenkopf (20) in einem maximal abgewinkelten Zustand zur Längsachse (36) des Instrumentenschafts (12), entsprechend einer maximalen Biegung der flexiblen Hohlwelle (42, 100, 200, 300, 400, 500, 600, 700, 800, 900, 1000), einen Winkel in einem Bereich von 60° bis 90° hat.

12. Chirurgisches Instrument nach einem der vorhergehenden Ansprüche, wobei die flexible Hohlwelle (42, 100, 200, 300, 400, 500, 600, 700, 800, 900, 1000) so ausgebildet ist, dass nebeneinanderliegende Federscheiben (54) in einem maximal abgewinkelten Zustand des Instrumentenkopfs (20) zur Längsachse (36) des Instrumentenschafts (12), entsprechend einer maximalen Biegung der flexiblen Hohlwelle (42, 100, 200, 300, 400, 500, 600, 700, 800, 900, 1000), an der bezüglich der Biegerichtung inneren Hohlwellenseite gerade in Anlage kommen oder noch voneinander beabstandet sind.

13. Chirurgisches Instrument nach einem der vorhergehenden Ansprüche, wobei die Schlitze (60) zwischen nebeneinanderliegenden Federscheiben (54) frei sind.

14. Chirurgisches Instrument nach einem der vorhergehenden Ansprüche, wobei entlang der Längsachse (36) des Instrumentenschafts (12) zumindest ein mechanisches und/oder elektrisches Verbindungselement (44) angeordnet und durch die flexible Hohlwelle hindurchgeführt ist und das mit dem Endeffektor verbindbar ist.

15. Chirurgisches Instrument nach einem der vorhergehenden Ansprüche, mit einem zum Instrumentenschaft (12) koaxialen Innenschaft (34) zum Abwinkeln des Instrumentenkopfs (20) mit Hilfe des Gelenks, wobei der Innenschaft (34) in Längsrichtung des Instrumentenschafts (12) verschiebbar ist und die Antriebswelle (32) gegenüber dem Instrumentenschaft (12) drehbar ist.
